# EUROPÄISCHE PATENTSCHRIFT

(11) **EP 3 640 383 B1**
(45) Veröffentlichungstag und Bekanntmachung des Hinweises auf die Patenterteilung: **30.11.2022**
(21) Anmeldenummer: 18200645.2
(22) Anmeldetag: 16.10.2018
(51) Int. Cl.: D04B 1/12, D04B 1/26

(54) **GESTRICKTEIL**
KNITTED PIECE
PIÈCE TRICOTÉE

(43) Veröffentlichungstag der Anmeldung: 22.04.2020
(73) Patentinhaber: medi GmbH & Co. KG, 95448 Bayreuth (DE)
(72) Erfinder: ATMANSPACHER, Jan, 95405 Warmensteinach (DE)
(74) Vertreter: Lindner Blaumeier Patent- und Rechtsanwälte

(56) Entgegenhaltungen:
- EP-A1- 1 668 998
- EP-A2- 0 854 219
- WO-A1-2016/097267
- DE-B3-102013 103 914
- US-B1- 7 441 419

## Beschreibung

Die Erfindung betrifft ein Gestrickteil, bestehend aus einem als Rundgestrick gestrickten Grundgestrick aus wenigstens einem Grundgestrickfaden.

Gestrickteile, beispielsweise in Form eines Bein- oder Armstrumpfes oder einer Bandage etc., werden häufig über ein Gelenk gezogen. Beim Abwinkeln des Gelenks kommt es in der Gelenkbeuge zu einem Zusammenschieben des Gestrickmaterials und zu einer Faltenbildung. Diese Faltenbildung beispielsweise im Bereich der Kniekehle oder der Armbeuge wird häufig als unangenehm empfunden, insbesondere, wenn über das Gestrickteil auch ein gewisser Druck auf das darunterliegende Gewebe ausgeübt werden soll, beispielsweise bei Ausführung des Gestrickteils als Kompressionsstrumpf.

Um dem zu begegnen, ist es bekannt, im Gelenkbereich respektive im Bereich der Gelenkbeuge eine Zone mit größerer Maschenweite bzw. größeren Maschen seitens des Grundgestricks vorzusehen, um dort das Fadenvolumen zu reduzieren und eine Art "Komfortzone" zu realisieren. Diese größere Maschenweite respektive Vergrößerung der Maschen selbst ist jedoch bereits in optischer Hinsicht nachteilig, als sich in diesem Bereich ein optisch stark gestörtes Maschen- oder Gestrickbild ergibt, da die deutlich größeren Maschen sichtbar sind und als eine Art Perforation wahrgenommen werden. Auch ändert sich durch dieses geänderte Maschenbild ein etwaiger zu erzeugender Druckgradient in Umfangs- und Längsrichtung des Gestrickteils gesehen, wie er beispielsweise bei Kompressionsgestrickteilen definiert vorgesehen wird. Denn durch die deutliche Vergrößerung der Maschen ergibt sich in diesem Bereich eine Druckabsenkung.

Der Erfindung liegt das Problem zu Grunde, ein demgegenüber verbessertes Gestrickteil anzugeben.

Zur Lösung dieses Problems ist es bei einem Gestrickteil der eingangs genannten Art erfindungsgemäß vorgesehen, dass wenigstens eine Zone mit reduziertem Fadenvolumen vorgesehen ist, die mittels mit ihren beiden Enden im Grundgestrick fixierten weiteren Fäden, von denen zumindest ein Teil dünner ist als der Grundgestrickfaden, gestrickt ist, wobei die Fäden anstelle des Grundgestrickfadens in mehreren separaten, sich in Umfangsrichtung um weniger als 360° erstreckenden, Maschen bildenden Reihen gestrickt sind.

Bei dem erfindungsgemäßen Gestrickteil erfolgt in wenigstens einer Zone eine gezielte, definierte Reduzierung des Fadenvolumens, sodass in dieser Zone, verglichen mit dem benachbarten Grundgestrickbereich, weniger "Fadenmasse" vorhanden ist. Dies geschieht, indem der quasi spiralförmig umlaufend gestrickte Grundgestrickfaden in dieser Zone lokal und, in Umfangsrichtung gesehen, partiell durch einen weiteren Faden ersetzt wird, wobei zumindest ein Teil dieser ersetzenden Fäden dünner ist als der Grundgestrickfaden. Der Grundgestrickfaden wird also in seinem spiralförmigen Umlauf lokal unterbrochen und stattdessen ein weiterer Faden eingestrickt, der seinerseits um weniger als 360° umläuft, bevorzugt um weniger als 270°, insbesondere um ca. 180° oder weniger, um eine lokale, quasi "ausgedünnte" Zone zu schaffen.

Der unterbrochene Grundgestrickfaden wie auch der weitere Faden ist jeweils im Grundgestrick respektive in dem die Zone umfassende Gestrickbereich fixiert respektive "verriegelt", mithin also fest eingebunden. Auf dieser Weise ist es möglich, in Umfangsrichtung gesehen quasi den Grundgestrickfaden und jeden der weiteren Fäden aneinander anschließend zu verstricken und einzubinden, während sie in Gestricklängsrichtung, also in Richtung der Maschenstäbchen Maschen bildend eingestrickt sind.

Je nachdem, wie viele weitere Fäden in wie vielen Maschenreihen, quasi ersetzend, eingestrickt werden, und wie weit die weiteren Fäden umlaufen, kann die Größe der eingestrickten Zone variiert werden. Da zumindest ein Teil der eingestrickten weiteren Fäden dünner ist als der Grundgestrickfaden, ergibt sich damit in dieser Zone respektive im Bereich dieser Teilreihen, in denen die weiteren Fäden nur partiell eingestrickt sind, zwangsläufig eine Reduzierung des Fadenvolumens, was zu einer verringerten Faltenbildung im Gelenkbereich führt und demzufolge das Tragen angenehmer gestaltet. Da sich die Maschenbildung als solche nicht ändert, da die Maschenweite bzw. Maschengröße gleichbleibt, sondern nur die Fadenstärke variiert wird, ergibt sich trotz Integration dieser partiellen Zone eine gleichbleibende Maschen- oder Gestrickoptik, so dass optisch gesehen dieser Bereich, sofern er nicht, worauf nachfolgend noch eingegangen wird, bewusst vorgehoben wird, auf den ersten Blick nicht erkennbar ist, gleichwohl jedoch die beschriebenen Vorteile insbesondere in Bezug auf die Faltenbildung aufweist. Auch kann, wenn es sich um ein Gestrickteil mit geringer oder stärkerer kompressiver Eigenschaft handelt, der Druckverlauf aufrechterhalten werden, da einerseits der Schussfaden, bei dem es sich bevorzugt um einen elastischen Schussfaden handelt, auch durch die mit den weiteren Fäden gestrickten Maschen durchläuft, mithin also durch die Zone umläuft, und da andererseits auch die eingestrickten weiteren Fäden eine entsprechende Elastizität aufweisen, beispielsweise vergleichbar mit der des Grundgestrickfadens, sodass sich in Bezug auf einen ausgeübten Kompressionsdruck im Bereich der Zone keine Änderung ergibt. Die Maschengröße, mit der die weiteren einzelnen Fäden verstrickt werden, ist also identisch zum restlichen Gestrick, das über den Grundgestrickfaden gestrickt ist, sie liegt im Bereich von ca. 1-3 mm, vorzugsweise ca. 1,8 mm, gesehen in Umfangsrichtung.

Wie beschrieben sind die weiteren, partiell eingestrickten Fäden, aber auch der im Bereich der Zone eingestrickte, von einem weiteren Faden abschnittsweise ersetzte Grundgestrickfaden im Gestrick fixiert. Dies erfolgt bevorzugt über Fang und/oder Flottung in Bezug auf die weiteren Fäden, die also über Fang und/oder Flottung im Grundgestrick fixiert sind. Der Grundgestrickfaden ist dann zwangsläufig über Fang und/oder Flottung in den über die weiteren Fäden gestrickten Maschen fixiert. Auf diese Weise wird eine stricktechnisch sichere Fixierung oder "Verriegelung" erreicht.

Gemäß einer ersten Erfindungsalternative können alle Teilreihen aus dem gleichen Faden gestrickt sein. Das heißt, dass jede partiell eingestrickte Maschenreihe, die eine Grundgestrickmaschenreihe lokal ersetzt, mit dem gleichen, verglichen mit dem Grundgestrickfaden dünneren Faden gestrickt ist.

Alternativ dazu ist es auch denkbar, dass eine oder mehrere Teilreihen mit einem ersten Faden und eine oder mehrere Teilreihen mit einem zweiten Faden gestrickt sind, wobei zumindest einer der beiden Fäden dünner als der Grundgestrickfaden ist. Bei dieser Erfindungsausgestaltung kommen also zwei oder mehr unterschiedliche weitere Fäden zum Einsatz, wobei zumindest einer dieser weiteren Fäden dünner ist als den Grundgestrickfaden, während der andere weitere Faden beispielsweise eine vergleichbare Dicke wie der Grundgestrickfaden oder sogar eine etwas höhere Dicke aufweisen kann, sich aber beispielsweise in der Farbe oder Elastizität oder dergleichen von den Eigenschaften des Grundgestrickfadens unterscheiden kann. Insgesamt ist, auch wenn zwei oder mehr unterschiedliche weitere Fäden verwendet werden, die Dickenauswahl der weiteren Fäden jedoch stets so zu treffen, dass sich in der Zone insgesamt ein niedrigeres Fadenvolumen ergibt, als wenn diese nur mit dem Grundgestrickfaden gestrickt ist.

Bevorzugt sind beide weiteren Fäden dünner als der Grundgestrickfaden, sodass jeder der beiden Fäden zu einer Reduzierung des Fadenvolumens beiträgt. Alternativ ist es aber auch denkbar, dass der eine Faden dünner und der andere Faden dicker als der Grundgestrickfaden ist, wobei sich jedoch insgesamt noch eine Fadenvolumenreduzierung gibt.

Die Teilreihen, gesehen in Richtung der Maschenstäbchen, können gemäß einer zweckmäßigen Erfindungsausgestaltung direkt aneinander anschließen. Das heißt, dass, gesehen in Gestricklängsrichtung respektive in Richtung der Maschenstäbchen, eine homogene, nur aus Teilreihen bestehende Zone vorgesehen ist, die außen herum von Grundgestrick umfasst ist. Bei den Teilreihen kann es sich um Reihen, die aus einem gleichen Faden gestrickt sind, handeln, oder um Reihen, die aus unterschiedlichen Fäden gestrickt sind, wie vorstehend beschrieben.

Alternativ hierzu ist es denkbar, dass nur jede x-te Reihe eine Teilreihe ist, wobei zwischen zwei Teilreihen eine oder mehrere Grundgestrickreihen liegen. Bei dieser Erfindungsausgestaltung ist, gesehen in Richtung der Maschenstäbchen respektive in Gestricklängsrichtung, das Grundgestrick nur abschnittsweise über eine oder mehrere Teilreihen unterbrochen, das heißt, dass der Grundgestrickfaden in der Zone abschnittsweise unterbrochen ist und wiederum in anderen Bereichen umläuft. Denkbar ist es beispielsweise die Grundgestrickreihen und die Teilreihen einander abzuwechseln, oder beispielsweise zwei Teilreihen mit einer dazwischen liegenden Grundgestrickreihe zu stricken, oder eine Teilreihe mit zwei dazwischen befindlichen Grundgestrickreihen, etc. die Variationsmöglichkeiten sind beliebig, wobei natürlich auch hier die Teilreihen aus einem einheitlich gleichen Faden gestrickt werden können, oder aus unterschiedlichen Fäden, wie vorstehend bereits beschrieben.

Erfindungsgemäß sind, wie beschrieben, die weiteren Fäden, zumindest zum Teil, dünner als der Grundgestrickfaden. Soweit die weiteren Fäden dünner als der Grundgestrickfaden sind, sollten sie wenigstens um den Faktor 0,9, besonders bevorzugt um den Faktor 0,8 dünner als der Grundgestrickfaden sein (Dicke Grundgestrickfaden x Faktor).

Die Feinheit der verwendeten Fäden respektive Garne wird beispielsweise wie in der Gütesicherung RAL-GZ387/1 "Medizinische Kompressionsstrümpfe", dort unter 2.7 "Feinheit von Garnen" in Verbindung mit dem Unterpunkt 4.4.3 "Verfahren zur Messung der Fadenstärke" beschriebenen Messverfahren bestimmt. Die Garnstärke der Teilreihen sollte, zumindest in jeder zweiten Reihe der gestrickten Zone, vorzugsweise größer 180 dtex gemäß der oben angegebenen RAL-GZ387/1 sein, soweit die weiteren Fäden betroffen sind. Denkbar ist es aber auch eine niedrigere dtex-Zahl.

Garnhersteller geben die Feinheit der Garne ebenfalls in der Einheit dtex an. Jedoch verwenden diese eine andere Messmethode. Ermittelt wird das Fadengewicht pro Längeneinheit bevorzugt nach der DIN 53830 oder ISO 2060. Grundsätzlich sollte das Fadengewicht pro Längeneinheit, gemessen nach den Herstellerangaben, der verwendeten weiteren Fäden ca. 10-30 dtex niedriger sein als das Fadengewicht des Grundgestrickfadens bzw. sollten weiteren Fäden von mindestens eine Stärke von 10 dtex, vorzugsweise ca. 22 dtex verwendet werden. Je größer die Differenz, umso eher kann sich auch eine Art transparentes Gestricks in der Zone im getragenen, also gespannten Zustand, verglichen zum Grundgestrick, ergeben.

Weiterhin ist es denkbar, dass die weiteren Fäden zumindest zum Teil eine höhere Elastizität als der Grundgestrickfaden aufweisen. Durch Verwendung von stärker elastischen weiteren Fäden kann, wenn erforderlich, eine Beibehaltung des Kompressionsdrucks erreicht werden, die sich möglicherweise aus einer Verringerung des Fadenvolumens ergibt. Durch die Verwendung von weiteren Fäden, die eine andere Elastizität aufweisen als den Grundgestrickfaden, kann einer etwaigen aus der Volumenverringerung resultierenden Druckvariation entgegengewirkt werden, sodass sich insgesamt ein gleichbleibender Druckverlauf auch innerhalb der Zone ergibt und es nicht zur Bildung einer Entlastungszone kommt. Das heißt, dass ein graduierter Kompressionsverlauf, wenn es sich bei dem Gestrickteil um ein kompressives Gestrickteil handelt, auch über die Zone hinweg einstellen respektive aufrechterhalten lässt, nachdem einerseits der elastische Schussfaden umläuft, also auch durch die Maschenreihen der weiteren Fäden läuft, andererseits aber auch über die Elastizität der weiteren Fäden hierauf Einfluss genommen werden kann.

Eine Weiterbildung sieht vor, als weitere Fäden solche zu verwenden, die zumindest zum Teil eine andere Farbe als der Grundgestrickfaden aufweisen. Auf dieser Weise kann optisch Einfluss genommen werden und entweder eine bewusste Markierung der Zone über die weiteren Fäden erreicht werden, oder eine entsprechende Variation aus Designgründen erfolgen.

Des Weiteren ist es möglich, in zumindest einem Teil der Teilreihen oder der Grundgestrickreihen der Zone einen Plüschfaden, der Plüschhenkel bildend eingestrickt ist, vorzusehen. In der Zone ist bevorzugt ein auf die Fäden mit reduzierte Fadenfeinheit, also die dünneren Fäden, aufplattierter Plüschfaden vorgesehen, der der Ausbildung von Plüschhenkel dient, über die wiederum eine Art Komfortzone, also eine praktisch weiche Zone gebildet wird. Dieser Plüschfaden kann nur auf die dünneren weiteren Fäden aufplattiert sein, er kann bei Bedarf aber auch auf den Grundgestrickfaden, soweit dieser durch die Zone läuft, aufplattiert sein. Diese Plüschfäden (gegebenenfalls als zusätzliche Fäden nur abschnittsweise umlaufend aufplattiert) ermöglichen also eine Verbesserung des Tragekomforts. Zwar erhöht sich hierdurch das Fadenvolumen wiederum etwas, gleichwohl kann diese Erhöhung einerseits über die gewählte Feinheit der weiteren Fäden wieder ausgeglichen sein, zum anderen ergibt sich wie beschrieben durch diese Plüschbildung eine beachtliche Komfortverbesserung, die auch beim Beugen des Gelenks spürbar ist.

Beim Gestrickteil kann es sich um ein handelsübliches Gestrickteil handeln, also einen üblichen Beinstrumpf oder dergleichen. Alternativ kann es sich auch um ein Kompressionsgestrick, insbesondere der Klassen 1 bis 4, handeln, wie es in der Gütesicherung RAL-GZ387/1 "Medizinische Kompressionsstrumpfe" definiert ist, handeln, wobei diese RAL in entsprechender Weise beispielsweise auf das Ausbilden von Armstrümpfen anzuwenden ist.

Bei dem Gestrickteil selbst kann es sich um einen Strumpf, sei es ein Arm- und Beinstrumpf, die Bandage oder einen Teil einer Orthese handeln.

Daneben betrifft die Erfindung ferner ein Verfahren zur Herstellung des, vorzugsweise einlagigen, Gestrickteils bestehend aus einem als Rundgestrick gestrickten Grundgestrick aus wenigstens einem Grundgestrickfaden und wenigstens einem eingelegten Schussfaden. Das Verfahren zeichnet sich dadurch aus, dass wenigstens eine Zone mit reduziertem Fadenvolumen erzeugt wird, die mittels mit ihren beiden Enden im Grundgestrick fixierten weiteren Fäden, von denen zumindest ein Teil dünner ist als der Grundgestrickfaden, gestrickt wird, wobei die Fäden anstelle des Grundgestrickfadens in mehreren separaten, sich in Umfangsrichtung um weniger als 360° erstreckenden, Maschen bildenden Reihen gestrickt werden.

An dieser Stelle expliziert der Hinweis, dass sämtliche Merkmale und Ausführungen, die vorstehend bezüglich des Gestrickteils gemacht wurden, in gleicher Weise auch für das erfindungsgemäße Verfahren gelten, so beispielsweise die Beschreibung in Bezug auf die Strickweise bezüglich der Abfolge der Teilreihen oder die Verwendung von elastischen oder gefärbten Fäden etc. Sämtliche Merkmale, die zu dem erfindungsgemäßen Gestrickteil ausgeführt wurden, sind in gleicher Weise als Verfahrensmerkmale offenbart.

Insgesamt zeichnet sich das Gestrickteil aus bzw. ermöglicht das erfindungsgemäße Verfahren die Bildung eines Gestrickteils, das eine spezifische, im Fadenvolumen reduzierte Komfortzone innerhalb des Grundgestricks aufweist. Es besteht lediglich, im einfachsten Fall, aus drei unterschiedlichen Fäden, nämlich wenigstens einem Grundgestrickfaden, wenigstens einem durchlaufenden, elastischen Schussfaden sowie einem weiteren Faden, wobei alternative aber auch zwei oder mehrere verschiedene Grundgestrickfäden, eine oder mehrere zusätzliche Schussfäden oder auch weitere zusätzliche Fäden unterschiedlicher Art verwendet werden können.

Als Fäden respektive Garne können beliebige Garne verwendet werden, mit oder ohne Seele, glatt oder texturiert. Die Fäden oder Garne können in ihrer Elastizität vergleichbar sein, sie können sich aber auch deutlich unterscheiden, so dass über die Verwendung entsprechend elastischer weiterer Fäden Einfluss auf die Elastizität innerhalb der Zone genommen werden kann, so dass die Elastizität innerhalb der Zone einerseits vom durchlaufenden Schussfaden, andererseits von dem oder den weiteren elastischen Fäden bestimmt wird, während die Elastizität außerhalb der Zone von dem Schussfaden und den Grundgestrickfaden bestimmt wird. Hierüber kann eine gezielte Einstellung der Elastizität in der Zone verglichen mit der Elastizität außerhalb der Zone erreicht werden, so dass ein gleichbleibender, homogener Druckverlauf über die Zone erreicht wird, sich mithin der Druck beispielsweise im Falle eines Kompressionsstrumpfes vom Grundgestrick zur Zone und wieder zum Grundgestrick nicht ändert. Er kann sich aber auch durch eine gezielte Elastizitätseinstellung innerhalb der Zone ändern.

Stricktechnisch wechseln in einem 360°-Umlauf in der Zone der Grundgestrickfaden und der weitere Faden einander ab. Beide sind bevorzugt über Fang und/oder Flottung im Gestrick verankert respektive fixiert. Der Grundgestrickfaden und der weitere Faden überlappen lediglich im Bereich nur eines oder mehrerer benachbarter Maschenstäbchen. Dabei ist der eine Faden Maschen bildend verstrickt, während der andere über Fang und/oder Flottung eingebunden ist, in benachbarten Abschnitt wiederum ist der andere Faden Maschen bildend verstrickt, während der erste Faden über Fang und/oder Flottung eingebunden ist.

Weitere Vorteile und Einzelheiten der Erfindung ergeben sich aus den folgenden Ausführungsbeispielen sowie den zugehörigen Zeichnungen. Hierbei zeigen schematisch:
- Fig. 1: eine Prinzipdarstellung eines erfindungsgemäßen Gestrickteils in einer Teilansicht mit einer nur aus gleichen weiteren Fäden gestrickten Zone innerhalb des Grundgestricks,
- Fig. 2: eine Ansicht entsprechend Fig. 1 mit einer Zone, in der in abwechselnden Reihen Grundgestrickfaden und weitere Fäden gestrickt sind,
- Fig. 3: eine Ansicht entsprechend Fig. 1, bei der in der Zone zwei verschiedene weitere Fäden einander abwechselnd gestrickt sind, wobei der eine Faden dünner als der Grundgestrickfaden ist und der andere weitere Faden dünner als der erste weitere Faden ist,
- Fig. 4: eine Ansicht entsprechend Fig. 1, wobei in der Zone ein erster weiterer Faden, der dünner als der Grundgestrickfaden ist, und ein zweiter weiterer Faden, der gleich dick oder geringzügig dicker als der Grundgestrickfaden ist, verstrickt sind, und
- Fig. 5: eine Ansicht entsprechend Fig. 1, wobei hier zusätzlich ein Plüschfaden aufplattiert ist.

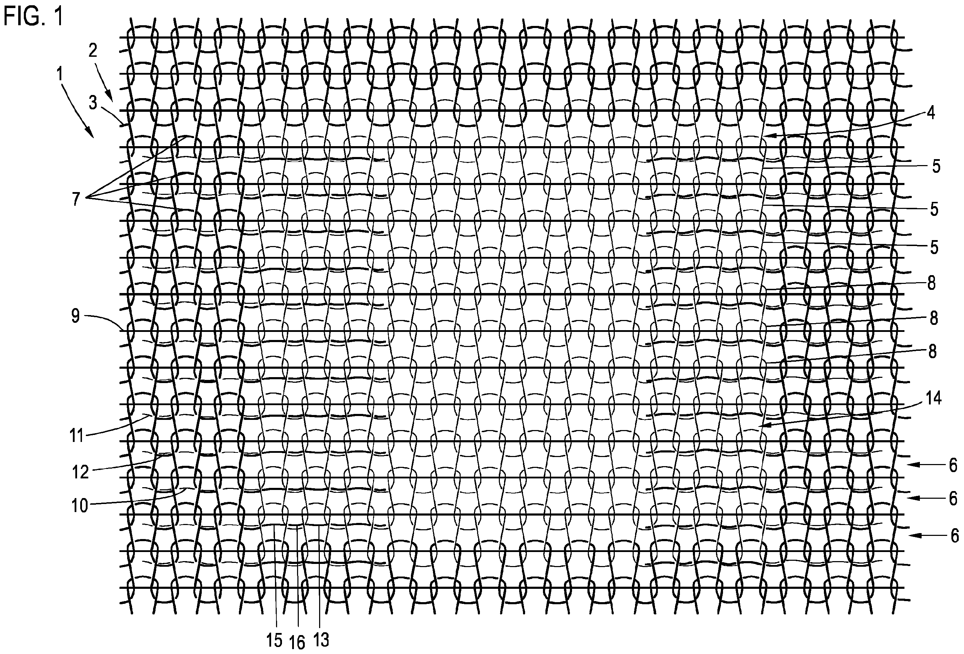

Fig. 1 zeigt ein erfindungsgemäßes Gestrickteil 1 in einem Ausschnitt. Bei dem Gestrickteil 1 handelt es sich beispielsweise um einen Strumpf, insbesondere einen Kompressionsstrumpf, das heißt, dass es sich bei dem Gestrick um ein Kompressionsgestrick handelt. Das Gestrickteil 1 besteht aus einem als Rundgestrick gestrickten Grundgestrick 2 aus wenigstens einem Grundgestrickfaden 3, der hier mit einer dicken schwarzen Linie dargestellt ist.

Ausgebildet ist eine Zone 4 innerhalb des Grundgestricks 2, in der das Fadenvolumen gegenüber dem Fadenvolumen im Grundgestrick 2 reduziert ist. Dies geschieht beim Ausführungsbeispiel gemäß Fig. 1 mittels mehrere separate weitere Fäden 5, die, jeweils Teilreihen 6 bildend, eingestrickt sind. Während im Grundgestrick 2 der Grunggestrickfaden 3 die Grundgestrickmaschen 7 bildet, bildet in der Zone 4 in jeder Teilreihe 6 jeder separate weitere Faden 5 die dortigen Maschen 8. Wie Fig. 1 zeigt, ersetzt demzufolge in jeder Reihe 6 der weitere Faden 5 den Grundgestrickfaden, der innerhalb des Grundgestricks 2 spiralförmig und die Maschen 7 bildend umläuft, jedoch in den jeweiligen Maschenreihen in der Zone 4 in den Reihen 6 unterbrochen ist und dort jeweils der weitere Faden 5 eingestrickt ist. Das heißt, dass demzufolge eine definierte Zone 4 innerhalb des Grundgestricks 2 gebildet wird, die sowohl in Umfangsrichtung um weniger als 360° umläuft, nachdem die Reihen 6 respektive die weiteren Fäden 5 um weniger als 360° laufen, und die auch in Richtung der Maschenstäbchen längenmäßig abgegrenzt ist.

Durch die Maschen 7 und 8 läuft ein ebenfalls spiralförmig umlaufende elastische Schussfaden 9, der den Gestrickteil 1 im gezeigten Beispiel seine wesentliche kompressive Eigenschaft verleiht.

Um das Fadenvolumen innerhalb der Zone 4 gegenüber dem Fadenvolumen in dem Grundgestrickbereich 2 zu verringern, ist jeder weitere Faden 5 in diesem Ausführungsbeispiel dünner als der Grundgestrickfaden 3, besitzt also eine höhere Feinheit respektive ein niedrigeres Garngewicht. Der jeweilige weitere Faden 5 ist demzufolge auch mittels einer dünneren Linie als der Grundgestrickfaden 3 dargestellt.

Wie beschrieben ist jeder separate Faden 5 um weniger als 360° umlaufend, bildet also eine nur partiell eingestrickte Reihe 6. Er hat demzufolge zwei Enden 10, mit denen er im anschließenden Grundgestrick 2 in derselben Reihe zu verankern ist. Dies geschieht, wie der Ausschnitt gemäß Fig. 1 zeigt, über Fang 11 und Flottung 12, mit denen jede weiterer Faden 6 mit den Enden 10 in Grundgestrick 2 eingebunden ist.

Zwangsläufig ist natürlich auch der Grundgestrickfaden 3 am Übergang zur Zone 4 unterbrochen und weist jeweils ein Ende 13 auf. Auch diese beiden Enden sind im Gestrick 14 der Zone 4, gebildet jeweils über die weiteren Fäden 5, über Fang 15 und Flottung 16 verankert, mithin also, wie auch jedes Ende 10, fest im Nachbargestrick "verriegelt".

Neben der geringeren Fadenstärke kann jeder weiterer Faden 5 auch eine andere Elastizität als der Grundgestrickfaden 3 besitzen, er kann beispielsweise weniger elastisch sein, um gegebenenfalls lokal die Kompression etwas zu erhöhen, oder elastischer, um sie lokal zu verringern, er kann aber auch die gleiche Elastizität aufweisen.

In jedem Fall führt die eingestrickte Zone 4 zu einer Komfortverbesserung. Am Gestrickteil 1, beispielsweise dem Strumpf, ist die Zone 4 an einer Position eingestrickt, die in der Tragestellung beispielsweise in die Kniekehle zu liegen kommt. Wird nun das Knie abgewinkelt, so kommt es zu einem Zusammenschieben des Gestrickteils 1 respektive der gestrickten Maschenreihen. Da in der Zone 4 in der Kniekehle das Fadenvolumen reduziert ist, schiebt sich dort zwangsläufig weniger "Fadenmasse" zusammen, so dass es zu einer geringeren und weniger unangenehmen Faltenbildung in diesem Bereich kommt. Gleichwohl bleibt, wenn die Elastizität der weiteren Fäden 5 entsprechend gewählt ist, ein etwaiger Druckgradient beispielsweise konstant oder wird in definiertem Maß variiert wird, um trotz reduzierten Fadenvolumens den Anforderungen gerecht zu werden.

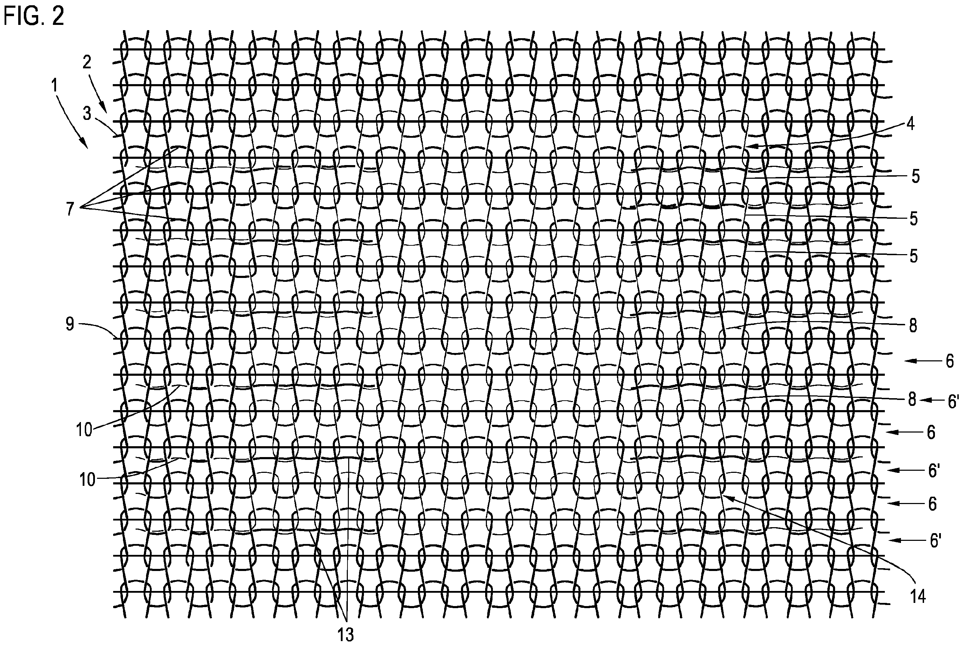

Fig. 2 zeigt ein weiteres Ausführungsbeispiel eines erfindungsgemäßen Gestrickteils 1, wobei hier für gleiche Elemente gleiche Bezugszeichen verwendet werden. Auch dieses besteht aus einem Grundgestrick 2 bestehend aus einem Grundgestrickfaden 3, der entsprechende Maschen 7 bildet. Vorgesehen ist es hier auch eine Zone 4 reduzierten Fadenvolumens, die auch hier über mehrere separate weitere Fäden 5, die lokal bzw. partiell eingestrickt sind, gebildet ist. Die weiteren Fäden 5 bilden wiederum entsprechende Maschen 8 und sind auch hier in lokalen Reihen 6 eingestrickt, wobei diese Reihen 6 in Richtung der Maschenstäbchen, die nach oben laufen, um jeweils eine Maschenreihe 6 versetzt zueinander angeordnet sind. Zwischen zwei Reihen 6 ist jeweils eine Reihe 6' gebildet aus dem Grundgestrickfaden, gestrickt. Das heißt, dass in der Zone 4 eine Reduzierung des Fadenvolumens gegeben ist, jedoch in geringerer Maße als im Beispiel gemäß Fig.1.

Auch hier ist natürlich der weitere Faden 5 dünner als der Grundgestrickfaden 3, weist also ein deutlich niedrigeres Fadengewicht respektive eine höhere Feinheit auf.

Die Verankerung der Enden 10 der einzelnen weiteren Fäden 5 wie auch der Enden 13 des Grundgestrickfadens 3 erfolgt auch hier über Fang und Flottung, ähnlich wie im Ausführungsbeispiel gemäß Fig. 1.

Der elastische Schussfaden 9, der sich spiralförmig durch das Grundgestrickteil zieht, läuft auch hier durch sämtliche Maschen 7, 8 sowohl des Grundgestricks 2 als auch der Zone 4. Alternativ ist es möglich, dass dieser nur in jeder n-ten Maschenreihe in das Gestrick eingelegt und/oder zumindest teilweise maschenbildend eingestrickt ist.

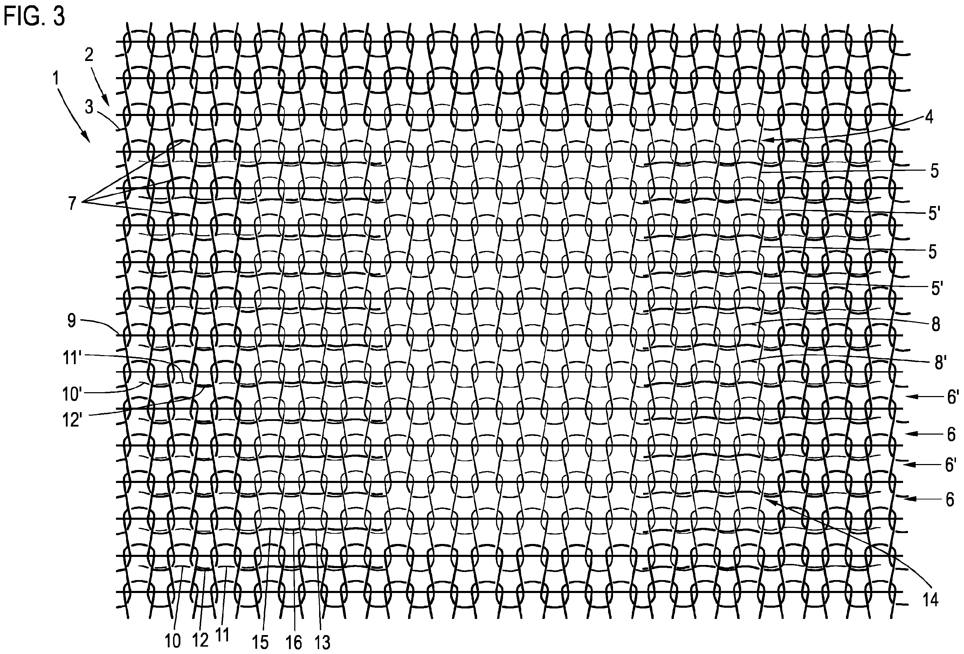

Fig. 3 zeigt ein Ausführungsbeispiel eines Gestrickteils 1 umfassend ein Grundgestrick 2 aus einem Grundgestrickfaden 3, der wiederum die Maschen 7 bildet, und aus einer Zone 4, die hier aus zwei unterschiedlichen weiteren Fäden 5, 5' gebildet ist. Die einzelnen Fäden 5 sind dünner als der Grundgestrickfaden 3, die einzelnen Fäden 5' sind nochmals dünner als die Fäden 5. Im gezeigten Beispiel bilden die Fäden 5 die Reihen 6, die jeweils um eine Maschenreihe 6', gebildet vom Faden 5', beabstandet sind, das heißt, dass die Fäden 5, 5' reihenweise einander abwechselt eingestrickt sind.

Die beiden Enden 10 und 10' der Fäden 5, 5' sind auch hier über Fang 11 und Flottung 12 bzw. 11' und 12' im Grundgestrick 2 verankert, während die Enden 13 des Grundgestrickfadens 3 in den jeweiligen Maschenreihen 6, 6' in ebenfalls bereits beschriebene Weise über Fang 15 und Flottung 16 verankert sind.

Auch hier ergibt sich eine Zone 4 deutlich reduzierten Fadenvolumens, wobei durch die Verwendung unterschiedlich dünner Fäden 5, 5' eine weitere Variationsmöglichkeit hinsichtlich der Ausbildung der Zone respektive reduzierendes Fadenvolumens gegeben ist. Da auch die Fäden 5, 5' in ihrer Elastizität variieren können, kann auch eine entsprechende Beeinflussung der gesamten Elastizität der Zone 4 und damit eine Beeinflussung der Elastizität des gesamten Gestrickteils 1 in diesem Bereich erfolgen.

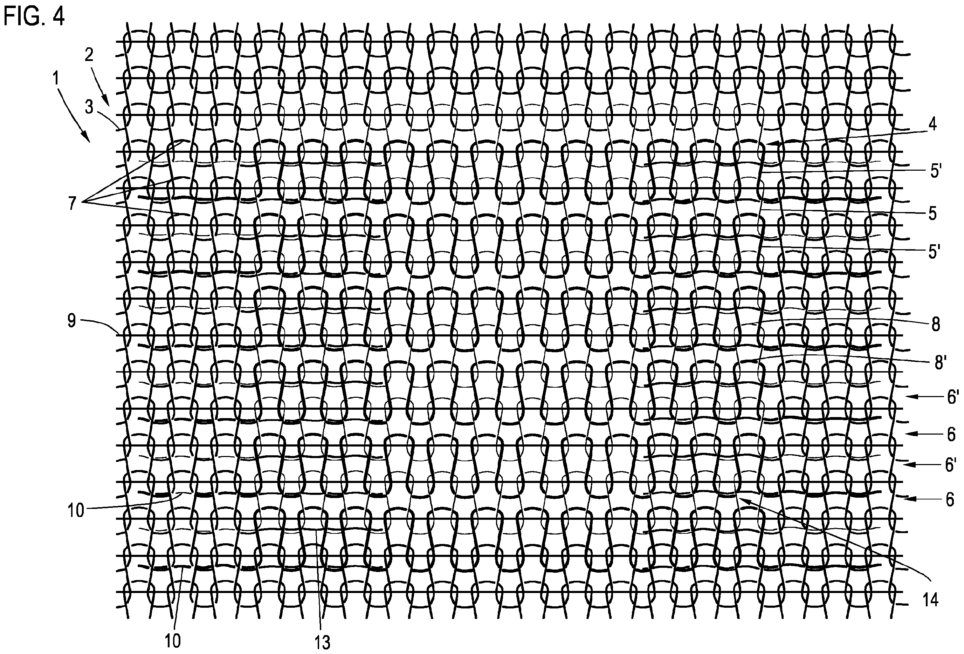

Fig. 4 zeigt eine Ausgestaltung eines Gestrickteils 1, die der aus Fig. 3 ähnlich ist. Auch hier ist ein Grundgestrick 2 aus einem Grundgestrickfaden 3, der Maschen 7 bildend gestrickt ist, vorgesehen, ebenso wie eine Zone 4, die auch hier aus zwei unterschiedlichen Fäden 5, 5', die abwechselnde Reihen 6, 6' bilden, gestrickt ist.

Bei dieser Ausgestaltung ist der Faden 5 sehr viel dünner als der Grundgestrickfaden 3 während der Faden 5' eine vergleichbare Dicke oder etwas größere Dicke als der Grundgestrickfaden 3 aufweist, jedoch beispielsweise in seiner Elastizität und/oder Farbigkeit in Vergleich zum Grundgestrickfaden 3 anders ist.

Es ergibt sich auch hier eine Reduzierung des Fadenvolumens, wenngleich nicht so stark wie im Ausführungsbeispiel wie gemäß Fig. 1 oder 2, jedoch kann über den in diesem Fall dickeren weiteren Faden 5' wiederum eine Einflussnahme auf die gesamte Elastizität oder die Farbigkeit etc. vorgenommen werden.

Auch hier läuft, ähnlich wie in Fig. 3, der elastische Schussfaden 9 durch sämtliche Maschen 7, die vom Grundgestrickfaden 3 gebildet werden, sowie die Maschen 8 und 8', die von den Fäden 5, 5' gebildet werden.

Ebenso sind natürlich auch hier die Enden 10, 10' der Fäden 5, 5' wie auch die Enden 13 des Grundgestrickfadens 3 über einen entsprechenden Fang und Flottung im jeweils benachbarten Gestrick verankert.

Als Fäden 3, 5, 5' können nahezu beliebige Fäden verwendet werden, die eine gewünschte Elastizität aufweisen, also eher stärker oder wenig stärker elastisch sind. Sie könne mit oder ohne Seele ausgeführt sein, es kann sich um glattes oder texturiertes Material handeln.

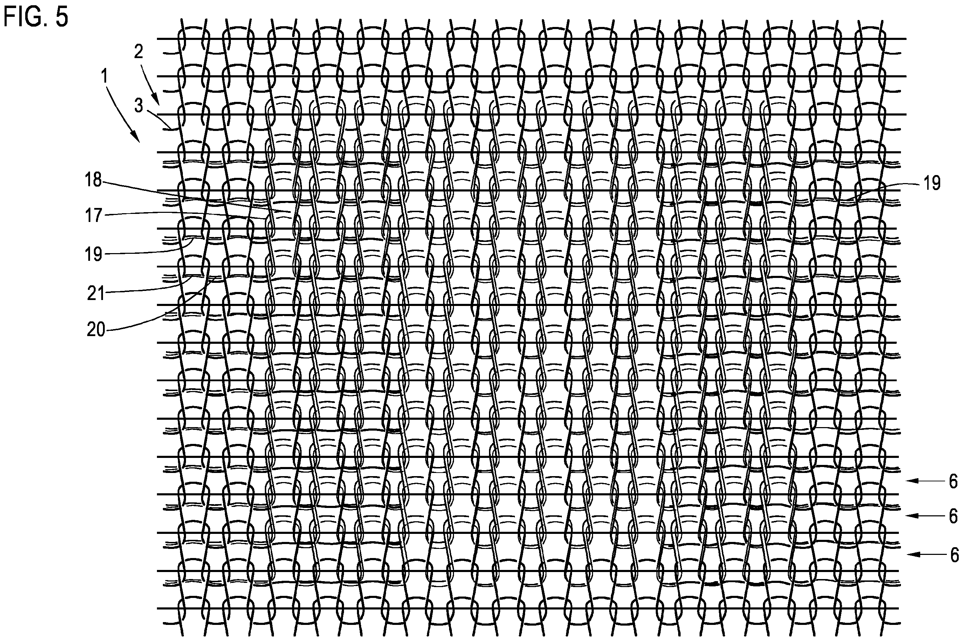

Fig. 5 zeigt schließlich ein Ausführungsbeispiel eines Gestrickteils 1, das dem Grunde nach dem aus Fig. 1 entspricht. Auch hier ist ein Grundgestrick 2 aus einem Grundgestrickfaden 3 sowie eine eingestrickte Zone 4 aus einem zusätzlichen dünneren Faden 5 vorgesehen, wobei diese Fäden 5 jeweils aufeinander folgende Teilreihen 6 bilden. Die Fadenenden 10 sind im Grundgestrick über Fang und Flottung in der selben Reihe verankert, wie auch die Enden 13 des Grundgestrickfadens im Gestrick der Zone 4 über Fang und Flottung verankert sind. Es wird auf die Ausführungen zu Fig. 1 verwiesen.

Hier ist jedoch zusätzlich ein Plüschfaden 17 in jeder Reihe 6 aus den Fäden 5 aufplattiert, der also mitgeführt wird und mit Plüschhenkeln 18 auf den Maschen 8 der Fäden 5 liegt. Hierüber kann eine weiche Komfortzone, die den Tragekomfort erhöht, gebildet werden. Der Plüschfaden 17 ist ebenfalls mit seinen beiden Enden 19 über Fang 20 und Flottung 21 im Grundgestrick in der selben Reihe verankert, parallel hier zum Faden 5 mit dessen Enden 10.

Ein solcher Plüschfaden 17 kann bei allen beschriebenen Beispielen verwendet werden, wobei er nur auf einem Teil oder allen Maschen 8, 8'der Fäden 5, 5' bzw. einem Teil der Reihen 6, 6'oder in allen Reihen 6, 6'aufplattiert sein kann, aber auch nur oder zusätzlich auf etwaigen Reihen 6'aus dem Grundgestrickfaden 3 innerhalb der Zone 4, wobei er natürlich auch außerhalt der Zone 4 auf dem Grundgestrickfaden 3 aufplattiert sein kann. Besonders bevorzugt ist der Faden derart auf den Grundgestrickfaden und/oder weiteren Faden aufplattiert, dass dieser einen sogenannten Sandwich-Plüsch ausbildet.

Entscheidend ist, dass in der Zone 4 ein reduzierendes Fadenvolumen im Vergleich zur benachbarten Grundgestrickzone gegeben ist. Der Übergang von der Zone 4 zum Bereich des Grundgestricks 3 ist fließend und nadelgenau, nachdem in den beiden benachbarten Maschenstäbchen jeweils eine Masche 7 des Grundgestricks 2 an einer Masche 8 bzw. 8' der Zone 4 anschließt und das jeweilige Fadenende 10, 10' bzw. 13 im jeweiligen benachbarten Gestrickbereich über Fang und Flottung ausläuft.

Je dünner die weiteren Fäden 5, 5' in der Zone 4 sind, umso transparenter kann dort im verglich zum Grundgestrick 2 das dortige Gestrick 14 gestrickt werden, was gegebenenfalls für die Optik vorteilhaft ist, da sich ein transparentes oder teiltransparentes Gestrick 14 ausbilden lässt.

Handelt es sich bei dem Gestrick 1 um ein Kompressionsgestrick, so kann trotz Integration der Zone 4, die dem Komfort dient, ein nahezu gleichbleibender Druckgradient sowohl in Umfangsrichtung als auch in Richtung der Maschenstäbchen erreicht werden, indem eine entsprechende Wahl der Elastizität der weiteren Fäden 8, 8' trotz ihrer deutlich reduzierten Dicke erreicht werden kann. Das heißt, dass sich weniger Fadenmaterial im Bereich der Gelenkbeuge befindet, bei nahezu gleicher Kompression.

Auch kann die Maschengröße in der Zone 4 nahezu identisch zur Maschengröße im Grundgestrick 2 sein, das heißt, dass sich, wenn gewünscht, rein optisch kein Zonenunterschied ergibt, sofern dieser nicht bewusst in Kauf genommen wird, beispielsweise durch Verwendung extrem dünner weitere Fäden 8, 8' oder durch Verwendung entsprechend eingefärbter Fäden 8, 8' etc.

## Patentansprüche

1. Gestrickteil, bestehend aus einem als Rundgestrick gestrickten Grundgestrick (2) aus wenigsten einem Grundgestrickfaden (3),wobei wenigstens eine Zone (4) mit reduziertem Fadenvolumen vorgesehen ist, **dadurch gekennzeichnet, dass** die Zone (4) mittels mit ihren beiden Enden (10, 10') in derselben Maschenreihe (6, 6') im Grundgestrick (2) fixierten weiteren Fäden (5, 5'), von denen zumindest ein Teil dünner ist als der Grundgestrickfaden (3), gestrickt ist, wobei die Fäden (5, 5') anstelle des unterbrochenen Grundgestrickfadens (3) in mehreren separaten, sich in Umfangsrichtung um weniger als 360° erstreckenden, Maschen (8, 8') bildenden Reihen (6, 6') gestrickt sind.

2. Gestrickteil nach Anspruch 1, **dadurch gekennzeichnet, dass** die Fäden (5, 5') über Fang (11, 11') und/oder Flottung (12, 12') im Grundgestrick (2) fixiert sind.

3. Gestrickteil nach Anspruch 1 oder 2, **dadurch gekennzeichnet, dass** eine oder mehrere separate Reihen (6) mit einem ersten Faden (5) und eine oder mehrere separate Reihen (6') mit einem zweiten Faden (6') gestrickt sind, wobei zumindest einer der beiden Fäden (6, 6') dünner als der Grundgestrickfaden (3) ist.

4. Gestrickteil nach Anspruch 3, **dadurch gekennzeichnet, dass** beide Fäden (6, 6') dünner als der Grundgestrickfaden (3) sind.

5. Gestrickteil nach Anspruch 3, **dadurch gekennzeichnet, dass** der eine Faden (6) dünner und der andere Faden (6') dicker als der Grundgestrickfaden (3) ist.

6. Gestrickteil nach einem der vorangehenden Ansprüche, **dadurch gekennzeichnet, dass** die separaten Reihen (6), gesehen in Richtung der Maschenstäbchen, direkt aneinander anschließen.

7. Gestrickteil nach einem der Ansprüche 1 bis 4, **dadurch gekennzeichnet, dass** nur jede x-te Reihe eine separate Reihe (6) ist, wobei zwischen zwei separaten Reihen (6) eine oder mehrere Grundgestrickreihen liegen.

8. Gestrickteil nach einem der vorangehenden Ansprüche, **dadurch gekennzeichnet, dass** die weiteren Fäden (6, 6'), soweit sie dünner sind als der Grundgestrickfaden (3), wenigstens um den Faktor 0,9, vorzugsweise um den Faktor 0,8 dünner als der Grundgestrickfaden sind.

9. Gestrickteil nach einem der vorangehenden Ansprüche, **dadurch gekennzeichnet, dass** die weiteren Fäden (6, 6') zumindest zum Teil eine andere, insbesondere höhere Elastizität als der Grundgestrickfaden (3) aufweisen.

10. Gestrickteil nach einem der vorangehenden Ansprüche, **dadurch gekennzeichnet, dass** die weiteren Fäden (6, 6') zumindest zum Teil eine andere Farbe als der Grundgestrickfaden (3) aufweisen.

11. Gestrickteil nach einem der vorangehenden Ansprüche, **dadurch gekennzeichnet, dass** in zumindest einem Teil der separaten Reihen (6, 6') der Zone ein Plüschfaden, der Plüschhenkel bildend eingestrickt ist, vorgesehen ist.

12. Gestrickteil nach einem der vorangehenden Ansprüche, **dadurch gekennzeichnet, dass** es ein Kompressionsgestrick ist.

13. Gestrickteil nach Anspruch 12, **dadurch gekennzeichnet, dass** in das Grundgestrick (2) wenigstens ein Schussfaden (9) eingelegt,oder eingelegt und verstrickt ist.

14. Gestrickteil nach einem der vorangehenden Ansprüche, **dadurch gekennzeichnet, dass** es ein Strumpf, eine Bandage oder ein Teil einer Orthese ist.

15. Verfahren zur Herstellung eines Gestrickteils (1) bestehend aus einem als Rundgestrick gestrickten Grundgestrick (2) aus wenigsten einem Grundgestrickfaden (3) und wenigstens einem eingelegten Schussfaden (9), wobei wenigstens eine Zone (4) mit reduziertem Fadenvolumen erzeugt wird, **dadurch gekennzeichnet, dass** die Zone (4) mittels mit ihren beiden Enden (10, 10') in derselben Maschenreihe (6, 6') im Grundgestrick (2) fixierten weiteren Fäden (5, 5'), von denen zumindest ein Teil dünner ist als der Grundgestrickfaden (3), gestrickt wird, wobei die Fäden (6, 6') anstelle des unterbrochenen Grundgestrickfadens (3) in mehreren separaten, sich in Umfangsrichtung um weniger als 360° erstreckenden, Maschen (8, 8') bildenden Reihen (6, 6') gestrickt werden.

## Claims

1. Knitted article, consisting of a ground knit (2) that is knitted in the form of a circular knit and is composed of at least one ground knit thread (3), wherein at least one zone (4) is provided with a reduced thread volume, **characterized in that** the zone (4) is knitted by means of further threads (5, 5'), which are fixed in place in the same stitch row (6, 6') in the ground knit (2) by way of their two ends (10, 10') and at least some of which are thinner than the ground knit thread (3), wherein the threads (5, 5'), instead of the interrupted ground knit thread (3), are knitted in multiple separate rows (6, 6') that form stitches (8, 8') and extend around less than 360° in a circumferential direction.

2. Knitted article according to Claim 1, **characterized in that** the threads (5, 5') are fixed in place in the ground knit (2) via tuck stitches (11, 11') and/or float stitches (12, 12').

3. Knitted article according to Claim 1 or 2, **characterized in that** one or more separate rows (6) are knitted with a first thread (5) and one or more separate rows (6') are knitted with a second thread (6'), wherein at least one of the two threads (6, 6') is thinner than the ground knit thread (3).

4. Knitted article according to Claim 3, **characterized in that** the two threads (6, 6') are thinner than the ground knit thread (3).

5. Knitted article according to Claim 3, **characterized in that** the one thread (6) is thinner and the other thread (6') is thicker than the ground knit thread (3).

6. Knitted article according to one of the preceding claims, **characterized in that** the separate rows (6) directly adjoin one another as seen in the direction of the stitch wale.

7. Knitted article according to one of Claims 1 to 4, **characterized in that** only every xth row is a separate row (6), wherein one or more ground knit rows lie between two separate rows (6).

8. Knitted article according to one of the preceding claims, **characterized in that** the further threads (6, 6'), if they are thinner than the ground knit thread (3), are thinner than the base knit thread by at least a factor of 0.9, preferably by a factor of 0.8.

9. Knitted article according to one of the preceding claims, **characterized in that** at least some of the further threads (6, 6') have a different, in particular higher, elasticity than the ground knit thread (3).

10. Knitted article according to one of the preceding claims, **characterized in that** at least some of the further threads (6, 6') have a different colour than the ground knit thread (3).

11. Knitted article according to one of the preceding claims, **characterized in that** a plush thread, which is knitted in to form plush loops, is provided in at least part of the separate rows (6, 6') of the zone.

12. Knitted article according to one of the preceding claims, **characterized in that** it is a compression knit.

13. Knitted article according to Claim 12, **characterized in that** at least one weft thread (9) is laid, or laid and knitted, into the ground knit (2).

14. Knitted article according to one of the preceding claims, **characterized in that** it is a stocking, a bandage or part of an orthosis.

15. Method for producing a knitted article (1) consisting of a ground knit (2) that is knitted in the form of a circular knit and is composed of at least one ground knit thread (3) and at least one laid-in weft thread (9), wherein at least one zone (4) is provided with a reduced volume of thread, **characterized in that** the zone (4) is knitted by means of further threads (5, 5'), which are fixed in place in the same stitch row (6, 6') in the ground knit (2) by way of their two ends (10, 10') and at least some of which are thinner than the ground knit thread (3), wherein the threads (6, 6'), instead of the interrupted ground knit thread (3), are knitted in multiple separate rows (6, 6') that form stitches (8, 8') and extend around less than 360° in a circumferential direction.

## Revendications

1. Pièce tricotée, comprenant un tricot de base (2) tricoté sous la forme d'un tricot circulaire et comprenant au moins un fil de tricot de base (3), au moins une zone (4) étant prévue qui possède un volume de fil réduit, **caractérisée en ce que** la zone (4) est tricotée au moyen d'autres fils (5, 5') qui sont fixés dans le tricot de base (2) par ses deux extrémités (10, 10') dans le même rang de mailles (6, 6') et dont au moins une partie est plus fine que le fil de tricot de base (3), les fils (5, 5') étant tricotés à la place du fil de tricot de base interrompu (3) en plusieurs rangs séparés (6, 6') qui forment des mailles (8, 8') qui s'étendent sur moins de 360° dans la direction circonférentielle.

2. Pièce tricotée selon la revendication 1, **caractérisée en ce que** les fils (5, 5') sont fixés dans le tricot de base (2) par guillochage (11, 11') et/ou flottage (12, 12').

3. Pièce tricotée selon la revendication 1 ou 2, **caractérisée en ce qu'**un ou plusieurs rangs séparés (6) sont tricotés avec un premier fil (5) et un ou plusieurs rangs séparés (6') sont tricotés avec un deuxième fil (6'), au moins un des deux fils (6, 6') étant plus fin que le fil de tricot de base (3).

4. Pièce tricotée selon la revendication 3, **caractérisée en ce que** les deux fils (6, 6') sont plus fins que le fil de tricot de base (3).

5. Pièce tricotée selon la revendication 3, **caractérisée en ce qu'**un fil (6) est plus fin, et l'autre fil (6') est plus gros, que le fil de tricot de base (3).

6. Pièce tricotée selon l'une des revendications précédentes, **caractérisée en ce que** les rangs séparés (6), vus dans la direction des colonnes de mailles, sont directement adjacents les uns aux autres.

7. Pièce tricotée selon l'une des revendications 1 à 4, **caractérisée en ce que** seulement un rang sur x est un rang séparé (6), un ou plusieurs rangs de tricot de base étant situés entre deux rangs séparés (6).

8. Pièce tricotée selon l'une des revendications précédentes, **caractérisée en ce que** les autres fils (6, 6'), dans la mesure où ils sont plus fins que le fil de tricot de base (3), sont plus fins d'au moins un facteur 0,9, de préférence d'un facteur 0,8, par rapport au fil de tricot de base.

9. Pièce tricotée selon l'une des revendications précédentes, **caractérisée en ce que** les autres fils (6, 6') présentent au moins en partie une élasticité différente, notamment plus élevée que le fil de tricot de base (3).

10. Pièce tricotée selon l'une des revendications précédentes, **caractérisée en ce que** les autres fils (6, 6') ont au moins en partie une couleur différente de celle du fil de tricot de base (3).

11. Pièce tricotée selon l'une des revendications précédentes, **caractérisée en ce qu'**un fil de peluche, qui est tricoté de façon à former des boucles de peluche, est prévu dans au moins une partie des rangs séparés (6, 6') de la zone.

12. Pièce tricotée selon l'une des revendications précédentes, **caractérisée en ce que** ladite pièce tricotée est un tricot à compression.

13. Pièce tricotée selon la revendication 12, **caractérisée en ce qu'**au moins un fil de trame (9) est inséré ou inséré et tricoté dans le tricot de base (2).

14. Pièce tricotée selon l'une des revendications précédentes, **caractérisée en ce que** ladite pièce tricotée est un bas, un bandage ou une partie d'une orthèse.

15. Procédé de réalisation d'une pièce tricotée (1) comprenant un tricot de base (2), tricoté sous la forme d'un tricot circulaire et comprenant au moins un fil de tricot de base (3) et au moins un fil de trame inséré (9), au moins une zone (4) étant générée qui possède un volume de fil réduit, **caractérisé en ce que** la zone (4) est tricotée au moyen d'autres fils (5, 5') qui sont fixés dans le tricot de base (2) par ses deux extrémités (10, 10') dans le même rang de mailles (6, 6') et dont au moins une partie est plus fine que le fil de tricot de base (3), les fils (6, 6') étant tricotés à la place du fil de tricot de base interrompu (3) en plusieurs rangs séparés (6, 6') qui forment des mailles (8, 8') qui s'étendent sur moins de 360° dans la direction circonférentielle.
